# EUROPEAN PATENT APPLICATION

(11) **EP 4 806 924 A1**
(43) Date of publication of application: **16.09.2026**
(21) Application number: 24890793.3
(22) Date of filing: 15.11.2024
(51) Int. Cl.: C07K 19/00, C12N 15/62, C12N 15/867, A61K 39/00, A61P 35/00

(54) **CHIMERIC ANTIGEN RECEPTOR AND USE THEREOF**

(30) Priority: 16.11.2023 CN 202311531216
(71) Applicant: Guangzhou Reforgene Medicine Co., Ltd., Guangzhou, Guangdong 510535 (CN)
(72) Inventor: LIANG, Junbin, Guangzhou, Guangdong 510535 (CN); MEI, Zhichao, Guangzhou, Guangdong 510535 (CN); XU, Hui, Guangzhou, Guangdong 510535 (CN); PAN, Yubin, Guangzhou, Guangdong 510535 (CN); ZENG, Xiaomin, Guangzhou, Guangdong 510535 (CN)
(74) Representative: Dai, Simin
(86) International application number: PCT/CN2024/132187
(87) International publication number: WO 2025/103439

(57) **Abstract**

Provided are a chimeric antigen receptor and use thereof. The chimeric antigen receptor comprises an extracellular domain, a transmembrane domain, and an intracellular domain which are connected in sequence; the extracellular domain recognizes and binds to antigens; the intracellular domain comprises an activation domain, or comprises a co-stimulatory domain and an activation domain; and in comparison to a parent intracellular domain, the lysine in the co-stimulatory domain and/or the activation domain is partially or fully replaced by glycine. The provided chimeric antigen receptor has an extended half-life and thus has strong tumor- or cancer-killing activity at a low effector-target ratio, increasing the anti-tumor activity of CAR-T against solid tumors and having broad clinical application prospects.

## Description

### CROSS-REFERENCE TO RELATED APPLICATIONS

This application claims priority to Chinese Patent Application No. 202311531216.6, filed November 16, 2023. The entire disclosures of the above Chinese patent application are incorporated herein by reference.

### TECHNICAL FIELD

The present disclosure relates to the field of biomedicine, and in particular to a chimeric antigen receptor and application thereof.

### BACKGROUND

Tumor immunotherapy is a therapeutic approach that restores the normal antitumor immune response of the body by restarting and maintaining the tumor-immunity cycle, thereby controlling and eliminating tumors. Tumor immunotherapy includes, for example, monoclonal antibody-based immune checkpoint inhibitors, therapeutic antibodies, cancer vaccines, cell therapies, and small molecule inhibitors. In recent years, tumor immunotherapy has advanced rapidly and has become the fourth major tumor treatment modality following surgery, radiotherapy, and chemotherapy. While immune checkpoint inhibitors, represented by anti-PD-1 monoclonal antibodies, have been approved for multiple tumor indications and continue to impact the current standard of care, cell therapies have demonstrated particularly strong efficacy in the field of hematologic malignancies. In the context of tumor immunotherapy, cell therapy refers to a therapeutic approach in which immune cells are expanded ex vivo in large numbers under the induction of cytokines to generate immune cells with high antitumor activity, and the expanded immune cells are then adoptively transferred into the patient to restore the patient's immune capacity and attack tumor cells. At present, several drugs based on CAR-T therapy have been approved for the treatment of hematologic malignancies, establishing the role of cell therapy in the treatment of various hematologic malignancies.

### SUMMARY

In a first aspect, the present disclosure provides a chimeric antigen receptor, wherein the chimeric antigen receptor comprises an extracellular domain, a transmembrane domain, and an intracellular domain connected in sequence; the extracellular domain recognizes and binds to an antigen; the intracellular domain comprises an activation domain, or comprises a costimulatory domain and an activation domain; and relative to a parental intracellular domain, lysines in the costimulatory domain and/or the activation domain are partially or completely substituted with glycines. In some embodiments, the number of the costimulatory domain is at least one.

In some embodiments, relative to the parental intracellular domain, lysines in the activation domain are partially or completely substituted with glycines.

In some embodiments, relative to the parental intracellular domain, lysines in at least one of the costimulatory domain and the activation domain are partially or completely substituted with glycines.

In some embodiments, relative to the parental intracellular domain, at least 1, at least 2, or at least 3 lysines in at least one of the costimulatory domain and the activation domain are substituted with glycines.

In some embodiments, relative to the parental intracellular domain, at least 1 lysine in at least one of the costimulatory domain and the activation domain is substituted with a glycine. In some embodiments, relative to the parental intracellular domain, at least 2 lysines in at least one of the costimulatory domain and the activation domain are substituted with glycines. In some embodiments, relative to the parental intracellular domain, at least 3 lysines in at least one of the costimulatory domain and the activation domain are substituted with glycines.

In some embodiments, relative to the parental intracellular domain, 1 lysine in one costimulatory domain is substituted with a glycine. In some embodiments, relative to the parental intracellular domain, 2 lysines in one costimulatory domain are substituted with glycines. In some embodiments, relative to the parental intracellular domain, 3 lysines in one costimulatory domain are substituted with glycines.

In some embodiments, relative to the parental intracellular domain, 1 lysine in one activation domain is substituted with a glycine. In some embodiments, relative to the parental intracellular domain, 2 lysines in one activation domain are substituted with glycines. In some embodiments, relative to the parental intracellular domain, 3 lysines in one activation domain are substituted with glycines.

In some embodiments, the parental intracellular domain consists of a native sequence. In some embodiments, the parental intracellular domain comprises a costimulatory domain derived from a native sequence and an activation domain derived from a native sequence. In some embodiments, the costimulatory domain of the parental intracellular domain is a native sequence. In some embodiments, the activation domain of the parental intracellular domain is a native sequence.

In some other embodiments, relative to the parental intracellular domain, lysines in at least one of the costimulatory domain and the activation domain are partially or completely substituted with glycines.

In some embodiments, the activation domain comprises a functional signaling domain of a protein selected from the group consisting of CD3ζ, CD3γ, CD3δ, CD3ε, FcεRIγ, FcRβ, CD79a, CD79b, FcγRIIa, DAP10, and DAP12.

In some specific embodiments, the activation domain is a functional signaling domain of CD3ζ.

In some examples, the activation domain comprises the amino acid sequence set forth in SEQ ID NO: 16.

In some embodiments, the costimulatory domain is a costimulatory domain of a protein selected from the group consisting of 4-1BB, CD27, CD28, OX-40, and ICOS.

In some specific embodiments, the costimulatory domain is a costimulatory domain of CD28 and/or 4-1BB.

In some examples, the costimulatory domain comprises the amino acid sequence set forth in SEQ ID NO: 12 and/or SEQ ID NO: 14.

In some embodiments, the transmembrane domain is a transmembrane domain of a protein selected from the group consisting of CD28, CD3e, CD45, CD4, CD5, CD8, CD9, CD16, CD22, CD33, CD37, CD64, CD80, CD86, CD134, CD137, and CD154.

In some specific embodiments, the transmembrane domain is a transmembrane domain of CD8.

In some examples, the transmembrane domain of the chimeric antigen receptor comprises the amino acid sequence set forth in SEQ ID NO: 10.

In some embodiments, the extracellular domain is an antibody or an antigen-binding fragment thereof, for example, an scFv, a single-domain antibody, or an F(ab)'.

In some embodiments, the chimeric antigen receptor further comprises a hinge region, wherein the hinge region connects the extracellular domain and the transmembrane domain.

In some embodiments, the hinge region comprises a hinge region of CD8α or CD28.

In some specific embodiments, the hinge region comprises a hinge region of CD8α.

In some examples, the hinge region comprises the amino acid sequence set forth in SEQ ID NO: 8.

In some embodiments, the chimeric antigen receptor further comprises a signal peptide.

In some specific embodiments, the signal peptide is a signal peptide of CD8α.

In some examples, the signal peptide comprises the amino acid sequence set forth in SEQ ID NO: 4.

In some embodiments, the extracellular domain targets a tumor antigen.

In some embodiments, the tumor antigen is selected from the group consisting of GPC3, CLDN18.2, GCC, EGFRvIII, ROR1, CLDN6, MSLN, ALPP, MUC1, LGR5, HER2, OR2H1, DLL-3, C-MET, glyco-cMET, glyco-LAMP1, CD123, CD33, CLL-1, CD70, CD38, FLT3, and GRP78.

In a second aspect, the present disclosure provides a polypeptide, wherein the polypeptide comprises the chimeric antigen receptor according to the first aspect.

In some embodiments, the polypeptide is linked to one or more additional functional molecules.

In some preferred embodiments, the additional functional molecule is selected from the group consisting of a signal peptide, a protein tag, other antigen-binding molecules, a cytokine, and combinations thereof.

In a third aspect, the present disclosure provides an isolated nucleic acid, wherein the nucleic acid encodes the chimeric antigen receptor according to the first aspect or the polypeptide according to the second aspect.

In some specific embodiments, the nucleic acid sequence encoding the transmembrane domain of the chimeric antigen receptor is set forth in SEQ ID NO: 9.

In some specific embodiments, the nucleic acid sequence encoding the activation domain of the chimeric antigen receptor is set forth in SEQ ID NO: 15.

In some specific embodiments, the nucleic acid sequence encoding the costimulatory domain of the chimeric antigen receptor is set forth in SEQ ID NO: 11 and/or SEQ ID NO: 13.

In some specific embodiments, the nucleic acid sequence encoding the hinge region of the chimeric antigen receptor is set forth in SEQ ID NO: 7.

In some specific embodiments, the nucleic acid sequence encoding the signal peptide of the chimeric antigen receptor is set forth in SEQ ID NO: 3.

In a fourth aspect, the present disclosure provides a vector, wherein the vector comprises the nucleic acid according to the third aspect.

In some embodiments, the vector is a plasmid, a cosmid, a bacteriophage, or a viral vector.

In some specific embodiments, the backbone of the viral vector is pHAGE.

In a fifth aspect, the present disclosure provides a gene expression cassette, wherein the gene expression cassette comprises a promoter and the nucleic acid according to the third aspect.

In a sixth aspect, the present disclosure provides an immune effector cell, wherein the immune effector cell expresses the chimeric antigen receptor according to the first aspect or the polypeptide according to the second aspect.

In some embodiments, the immune effector cell is selected from one or more of T cells, NK cells, NKT cells, monocytes, mast cells, macrophages, dendritic cells, CIK cells, and stem cell-derived immune effector cells.

In some specific embodiments, the immune effector cell is a T cell, an NK cell, or an NKT cell; for example, a T cell.

In a seventh aspect, the present disclosure provides a method for preparing an immune effector cell, wherein the method comprises: introducing the vector according to the fourth aspect or the gene expression cassette according to the fifth aspect into a parental immune effector cell, thereby obtaining the immune effector cell.

In an eighth aspect, the present disclosure provides a pharmaceutical composition, wherein the pharmaceutical composition comprises the chimeric antigen receptor according to the first aspect, the polypeptide according to the second aspect, the nucleic acid according to the third aspect, the vector according to the fourth aspect, the gene expression cassette according to the fifth aspect, and/or the immune effector cell according to the sixth aspect.

In some embodiments, the pharmaceutical composition further comprises a pharmaceutically acceptable carrier and/or excipient.

In the present disclosure, the pharmaceutical composition may comprise a buffer such as neutral buffered saline or sulfate-buffered saline; a carbohydrate such as glucose, mannose, sucrose, dextran, or mannitol; a protein; a polypeptide or an amino acid such as glycine; an antioxidant; a chelating agent such as EDTA or glutathione; an adjuvant (for example, aluminum hydroxide); and a preservative.

In a ninth aspect, the present disclosure provides the use of the chimeric antigen receptor according to the first aspect, the polypeptide according to the second aspect, the nucleic acid according to the third aspect, the vector according to the fourth aspect, the gene expression cassette according to the fifth aspect, the immune effector cell according to the sixth aspect, and/or the pharmaceutical composition according to the eighth aspect, in the preparation of a medicament for diagnosing, preventing, and/or treating a tumor or cancer, wherein the tumor comprises a solid tumor and a non-solid tumor.

In some embodiments, the solid tumor is selected from the group consisting of liver cancer, gastric cancer, lung cancer, breast cancer, head and neck cancer, bladder cancer, ovarian cancer, cervical cancer, kidney cancer, pancreatic cancer, liposarcoma, melanoma, adrenal cancer, schwannoma, malignant fibrous histiocytoma, and esophageal cancer; and the non-solid tumor is selected from the group consisting of B-cell lymphoma, Hodgkin's lymphoma, chronic myeloid leukemia, and acute myeloid leukemia.

In a tenth aspect, the present disclosure provides a method of diagnosing, preventing, and/or treating a tumor or cancer in a patient in need thereof, wherein the method comprises administering to the patient in need thereof an effective amount of the chimeric antigen receptor according to the first aspect, the polypeptide according to the second aspect, the nucleic acid according to the third aspect, the vector according to the fourth aspect, the gene expression cassette according to the fifth aspect, the immune effector cell according to the sixth aspect, and/or the pharmaceutical composition according to the eighth aspect, wherein the tumor comprises a solid tumor and a non-solid tumor.

The pharmaceutical composition of the present disclosure may be administered in a manner suitable for diagnosing, preventing, and/or treating a tumor or cancer. The amount and frequency of administration are determined by factors such as the patient's condition and the type and severity of the patient's disease. Administration may be carried out in any convenient manner, including by spraying, injection, swallowing, infusion, implantation, or transplantation. The compositions described herein may be administered to a patient subcutaneously, intradermally, intratumorally, intranodally, intrathecally, intramuscularly, by intravenous injection, or intraperitoneally.

In some embodiments, the solid tumor is selected from the group consisting of liver cancer, gastric cancer, lung cancer, breast cancer, head and neck cancer, bladder cancer, ovarian cancer, cervical cancer, kidney cancer, pancreatic cancer, liposarcoma, melanoma, adrenal cancer, schwannoma, malignant fibrous histiocytoma, and esophageal cancer; and the non-solid tumor is selected from the group consisting of B-cell lymphoma, Hodgkin's lymphoma, chronic myeloid leukemia, and acute myeloid leukemia.

In an eleventh aspect, the present disclosure provides a composition for diagnosing, preventing, and/or treating a tumor or cancer, wherein the composition comprises the chimeric antigen receptor according to the first aspect, the polypeptide according to the second aspect, the nucleic acid according to the third aspect, the vector according to the fourth aspect, the gene expression cassette according to the fifth aspect, the immune effector cell according to the sixth aspect, and/or the pharmaceutical composition according to the eighth aspect, wherein the tumor comprises a solid tumor and a non-solid tumor.

In some embodiments, the solid tumor is selected from the group consisting of liver cancer, gastric cancer, lung cancer, breast cancer, head and neck cancer, bladder cancer, ovarian cancer, cervical cancer, kidney cancer, pancreatic cancer, liposarcoma, melanoma, adrenal cancer, schwannoma, malignant fibrous histiocytoma, and esophageal cancer; and the non-solid tumor is selected from the group consisting of B-cell lymphoma, Hodgkin's lymphoma, chronic myeloid leukemia, and acute myeloid leukemia.

On the basis of common knowledge in the art, the above preferred conditions may be combined in any manner to obtain preferred examples of the present disclosure.

The reagents and starting materials used in the present disclosure are all commercially available.

In some embodiments, the chimeric antigen receptor has an extended half-life and exhibits strong tumor-killing activity even at a low effector-to-target ratio, which contributes to improving the antitumor activity of CAR-T against solid tumors, and has broad prospects for clinical application.

### BRIEF DESCRIPTION OF THE DRAWINGS

FIG. 1 is a graph showing the surface CAR expression levels of CAR-T cells as detected by flow cytometry in Example 3.
FIG. 2 is a graph showing the killing effect of CAR-T-1, CAR-T-2, CAR-T-3, CAR-T-4, and CAR-T-5 cells against target cells in Example 4.
FIG. 3 is a graph showing in vivo imaging of tumor-bearing mice in Example 5.
FIG. 4 is a graph showing the killing effect of CAR-T-GC33-1 and CAR-T-1-B1-1 cells against target cells in Example 7.
FIG. 5 is a graph showing the killing effect of CAR-T-GC33-2 and CAR-T-1-B1-2 cells against target cells in Example 9.

### DETAILED DESCRIPTION OF THE EMBODIMENTS

### Definitions of Terms

As used herein, the term "chimeric antigen receptor" or "CAR" generally refers to a fusion protein comprising an extracellular domain capable of binding to an antigen and an intracellular domain. The CAR is the core component of a chimeric antigen receptor immune effector cell, and may comprise an antigen (for example, tumor-associated antigen, TAA) binding domain, a transmembrane domain, and an intracellular domain. In the present disclosure, the chimeric antigen receptor may combine antibody-based antigen (for example, GPC3) specificity with an immune effector cell receptor activation intracellular domain. Immune effector cells genetically modified to express the CAR are capable of specifically recognizing and eliminating malignant cells expressing the target antigen.

As used herein, the term "extracellular domain" generally refers to a domain capable of specifically binding to an antigen, including but not limited to a single-chain antibody, an antibody or an antigen-binding fragment thereof, a tethered ligand, or the extracellular domain of a co-receptor. For example, the extracellular domain may comprise an antibody or an antigen-binding fragment thereof that specifically binds to an antigen expressed on a cell. As used in the present disclosure, the terms "binding domain," "extracellular domain," "extracellular binding domain," "antigen-specific binding domain," and "extracellular antigen-specific binding domain" are used interchangeably, and provide a domain or fragment of a CAR that is capable of specifically binding to a target antigen (for example, GPC3). The extracellular domain may be of natural origin, synthetic origin, semi-synthetic origin, or recombinant origin. The term "specific binding" generally refers to a measurable and reproducible interaction, such as the binding between an antigen and an antibody, which can determine the presence of a target in the presence of a heterogeneous population of molecules (including biomolecules). For example, an antibody that specifically binds an antigen (which may be an epitope) is an antibody that binds the antigen with greater affinity, avidity, more readily, and/or with greater duration than it binds other antigens. As used herein, the term "epitope" and other grammatical forms thereof may refer to a portion of an antigen that can be recognized by an antibody, a B cell, a T cell, or an engineered cell. For example, the epitope may be a tumor epitope or a pathogen epitope recognized by a chimeric antigen receptor. Multiple epitopes within an antigen may also be recognized. An epitope may also be mutated.

As used herein, the term "antibody" is used in its broadest sense and refers to a polypeptide or a combination of polypeptides that comprises sufficient sequence from the variable region of an immunoglobulin heavy chain and/or sufficient sequence from the variable region of an immunoglobulin light chain to be capable of specifically binding to an antigen. As used herein, the term "antibody" encompasses various forms and various structures, as long as they exhibit the desired antigen-binding activity. In the present disclosure, unless the context clearly dictates otherwise, the term "antibody" includes not only intact antibodies but also antigen-binding fragments of antibodies. As used herein, the term "antibody" includes alternative protein scaffolds or artificial scaffolds having grafted complementarity-determining regions (CDRs) or CDR derivatives. Such scaffolds include antibody-derived scaffolds (which comprise mutations introduced, for example, to stabilize the three-dimensional structure of the antibody) as well as synthetic scaffolds comprising, for example, biocompatible polymers. See, for example, Korndorfer et al., 2003, Proteins: Structure, Function, and Bioinformatics, 53(1):121-129 (2003); Roque et al., Biotechnol. Prog. 20:639-654 (2004). Such scaffolds may also include non-antibody-derived scaffolds, for example, scaffold proteins known in the art to be useful for grafting CDRs, including but not limited to tenascin, fibronectin, peptide aptamers, and the like.

As used herein, the term "antigen-binding fragment" refers to a fragment that does not have the entire structure of a complete antibody and comprises only a portion or a variant of a portion of a complete antibody, wherein the portion or variant of the portion is capable of binding to an antigen. By way of example, as used herein, the term "antigen-binding fragment" includes, but is not limited to, Fab, F(ab')2, Fab', Fab'-SH, Fd, Fv, scFv, diabody, and single-domain antibody. As used herein, the terms "single-domain antibody (sdAb)," "VHH domain," and "nanobody" have the same meaning and are used interchangeably, and refer to a cloned variable region of a heavy-chain antibody that is constructed as a single-domain antibody consisting of only one heavy-chain variable region, which is the smallest antigen-binding fragment having complete function. Typically, a heavy-chain antibody that naturally lacks a light chain and heavy chain constant region 1 (CH1) is first obtained, and then the variable region of the antibody heavy chain is cloned to construct a single-domain antibody consisting of only one heavy-chain variable region. It should be noted that nanobodies may be used to form other antibody formats; for example, an antibody may comprise VH-CH2-CH3 from N-terminus to C-terminus, or may comprise VH-CH1-CH2-CH3; and may form a homodimer, for example, a heavy-chain dimeric antibody having no light chains.

As used herein, the term "antibody" may be derived from any animal, including but not limited to humans and non-human animals, wherein the non-human animals may be selected from primates, mammals, rodents, and vertebrates, for example, camelids, llamas, guanacos, alpacas, sheep, rabbits, mice, rats, or chondrichthyes (for example, sharks).

As used herein, the term "transmembrane domain" refers to a structure capable of anchoring the CAR to the plasma membrane of a cell, which is connected to the intracellular domain and functions to transmit signals. The native transmembrane portions of common cell adhesion molecules may be used as the "transmembrane domain" of a CAR. For example, the native transmembrane portion of CD8α or the native transmembrane portion of CD28 may be used as the "transmembrane domain" in the CAR.

As used herein, the term "activation domain" refers to a molecule on an immune effector cell that specifically binds to an associated stimulatory ligand present on an antigen-presenting cell.

As used herein, the term "costimulatory domain" refers to an associated binding partner on an immune effector cell that specifically binds to a costimulatory ligand, thereby mediating a costimulatory response of the immune cell, such as, but not limited to, proliferation.

As used herein, the term "target" refers to specific binding, and in particular to a non-random binding reaction between two molecules, such as the reaction between an antibody and the antigen to which it is directed. In certain embodiments, an antibody that specifically binds a certain antigen (or an antibody that is specific for a certain antigen) refers to an antibody that binds the antigen with an affinity (KD) of less than about 10-5 M, for example, less than about 10-6 M, 10-7 M, 10-8 M, 10-9 M, or 10-10 M or less.

As used herein, the term "tumor" or "cancer" refers to all neoplastic cell growth and proliferation, whether malignant or benign, and all pre-cancerous and cancerous cells and tissues.

As used herein, the term "tumor antigen" refers to a tumor-associated antigen (TAA) or a tumorspecific antigen (TSA). A TAA or TSA may be expressed on hematologic cancer cells. A TAA or TSA may be expressed on solid tumor cells. The tumor antigen is selected from one or more of the following: CD19, CD133, CD123, CD20, CD22, CD30, CD33, CD171, CD80/86, CA125, C-met, L1CAM, EC, DLL3, CD99, GRP78, 5T4, CD138, CS-1 (also known as CD2 subclass 1, CRACC, SLAMF7, CD319, or 19A24), glypican-3 (GPC3), claudin 18.2, guanylate cyclase C (GCC/GUCY2C), mesothelin (MSLN), epidermal growth factor receptor (EGFR), prostatespecific membrane antigen (PSMA), carcinoembryonic antigen (CEA), epithelial cell adhesion molecule (EpCAM), alpha-fetoprotein (AFP), tyrosine-protein kinase receptor UFO (AXL), death receptor 5 (DR5), NKG2D ligands, prostate stem cell antigen (PSCA), macrophage-stimulating protein receptor (MST1R), leukocyte immunoglobulin-like receptor B4 (LILRB4), C-type lectinlike molecule-1 (CLL-1 or CLECL1), epidermal growth factor receptor variant III (EGFRvIII), ganglioside GD2, ganglioside GD3, B-cell maturation antigen (BCMA, a member of the TNF receptor family), Tn antigen (for example, Tn Ag or GalNAcα-Ser/Thr), glyco-cMET, glyco-LAMP1, receptor tyrosine kinase-like orphan receptor 1 (ROR1), Fms-like tyrosine kinase 3 (FLT3); tumor-associated glycoprotein 72 (TAG72), CD38, CD44v6, B7H3 (CD276), B7-H4, KIT (CD117), interleukin-13 receptor subunit alpha-2 (IL-13Ra2 or CD213A2), interleukin-11 receptor alpha (IL-11Ra), prostate stem cell antigen (PSCA), protease serine 21, vascular endothelial growth factor receptor 2 (VEGFR2), Lewis(Y) antigen, CD24, platelet-derived growth factor receptor beta (PDGFR-β), stage-specific embryonic antigen-4 (SSEA-4), folate receptor alpha (FR-α), receptor tyrosine-protein kinase ERBB2 (Her2/neu), cell surface-associated mucin 1 (MUC1), cell surface-associated mucin 16 (MUC16), epidermal growth factor receptor (EGFR), neural cell adhesion molecule (NCAM), prostase, prostatic acid phosphatase (PAP), mutated elongation factor 2 (ELF2M), ephrin B2, fibroblast activation protein alpha (FAP), insulin-like growth factor 1 receptor (IGF-I receptor), carbonic anhydrase IX (CAIX), proteasome (for example, proteasome or macropain factor) subunit beta type 9 (LMP2), glycoprotein 100 (gp100), an oncogene fusion protein (bcr-abl) consisting of breakpoint cluster region (BCR) and Abelson murine leukemia viral oncogene homolog 1 (Abl), tyrosinase, ephrin type-A receptor 2 (EphA2), fucosyl GM1; sialyl Lewis adhesion molecule (sLe), transglutaminase 5 (TGS5), high molecular weight melanoma-associated antigen (HMWMAA), o-acetyl-GD2 ganglioside (OAcGD2), folate receptor beta, tumor endothelial marker 1 (TEM1/CD248), tumor endothelial marker 7-related (TEM7R), claudin 6 (CLDN6), thyroid-stimulating hormone receptor (TSHR), G protein-coupled receptor class C group 5 member D (GPRC5D), chromosome X open reading frame 61 (CXORF61), CD97, CD179a, anaplastic lymphoma kinase (ALK), polysialic acid, placentaspecific 1 (PLAC1), hexose portion of globoH glycoceramide (GloboH), mammary gland differentiation antigen (NY-BR-1), urothelial differentiation-specific glycoprotein (uroplakin) 2 (UPK2), hepatitis A virus cellular receptor 1 (HAVCR1), adrenergic receptor beta 3 (ADRB3), pannexin 3 (PANX3), G protein-coupled receptor 20 (GPR20), lymphocyte antigen 6 complex locus K9 (LY6K), olfactory receptor 51E2 (OR51E2), TCR gamma alternate reading frame protein (TARP), Wilms tumor protein (WT1); cancer/testis antigen 1 (NY-ESO-1), cancer/testis antigen 2 (LAGE-1a), melanoma-associated antigen 1 (MAGE-A1), ETS translocation variant gene 6 located on chromosome 12p (ETV6-AML), sperm protein 17 (SPA17), X antigen family member 1A (XAGE1), angiopoietin-binding cell surface receptor 2 (Tie 2), melanoma cancer testis antigen-1 (MAD-CT-1), melanoma cancer testis antigen-2 (MAD-CT-2), Fos-related antigen 1, p53, p53 mutants, prostein, prostate cancer tumor antigen-1 (PCTA-1 or galectin 8), melanoma antigen 1 recognized by T cells (MelanA or MART1); rat sarcoma (Ras) mutants, human telomerase reverse transcriptase (hTERT), melanoma inhibitor of apoptosis protein (ML-IAP), ERG (transmembrane protease serine 2 (TMPRSS2) ETS fusion gene), N-acetylglucosaminyltransferase V (NA17), paired box protein Pax-3 (PAX3), androgen receptor, cyclin B1, v-myc avian myelocytomatosis viral oncogene neuroblastoma-derived homolog (MYCN), Ras homolog family member C (RhoC), tyrosinase-related protein 2 (TRP-2), cytochrome P450 1B1 (CYP1B1), squamous cell carcinoma antigen 3 recognized by T cells (SART3), paired box protein Pax-5 (PAX5), proacrosin-binding protein sp32 (OY-TES1), lymphocyte-specific protein tyrosine kinase (LCK), A-kinase anchor protein 4 (AKAP-4), synovial sarcoma X breakpoint 2 (SSX2), receptor for advanced glycation end products (RAGE-1), legumain, human papillomavirus E6 (HPV E6), human papillomavirus E7 (HPV E7), intestinal carboxyl esterase, mutated heat shock protein 70-2 (mut hsp70-2), CD79a, CD79b, CD72, leukocyte-associated immunoglobulin-like receptor 1 (LAIR1), Fc fragment of IgA receptor (FCAR or CD890), leukocyte immunoglobulin-like receptor subfamily A member 2 (LILRA2), CD300 molecule-like family member f (CD300LF), C-type lectin domain family 12 member A (CLEC12A), bone marrow stromal cell antigen 2 (BST2), EGF-like module-containing mucin-like hormone receptor-like 2 (EMR2), lymphocyte antigen 75 (LY75), placental alkaline phosphatase (ALPP), Fc receptor-like 5 (FCRL5), and/or immunoglobulin lambda-like polypeptide 1, leucinerich repeat-containing G-protein-coupled receptor 5 (LGR5), and olfactory receptor OR2H.

As used herein, the term "solid tumor" refers to a tumor selected from the group consisting of liver cancer, gastric cancer, lung cancer, breast cancer, colon cancer, renal cell carcinoma, non-small cell lung cancer, small intestine cancer, esophageal cancer, bone cancer, pancreatic cancer, skin cancer, head and neck cancer, cutaneous or intraocular malignant melanoma, liposarcoma, melanoma, uterine cancer, ovarian cancer, rectal cancer, cancer of the anal region, testicular cancer, fallopian tube cancer, endometrial cancer, cervical cancer, vaginal cancer, vulvar cancer, Hodgkin's disease, non-Hodgkin's lymphoma, cancer of the endocrine system, thyroid cancer, parathyroid cancer, adrenal cancer, schwannoma, malignant fibrous histiocytoma, soft tissue sarcoma, urethral cancer, penile cancer, solid tumors of childhood, bladder cancer, cancer of the kidney or ureter, renal pelvis cancer, central nervous system (CNS) tumors, spinal axis tumors, brainstem glioma, pituitary adenoma, Kaposi's sarcoma, epidermoid carcinoma, and squamous cell carcinoma.

As used herein, the term "non-solid tumor" refers to a tumor selected from the group consisting of chronic lymphocytic leukemia (CLL), acute leukemia, acute lymphoid leukemia (ALL), B-cell acute lymphoid leukemia (B-ALL), T-cell acute lymphoid leukemia (T-ALL), chronic myeloid leukemia (CML), acute myeloid leukemia (AML), B-cell prolymphocytic leukemia, blastic plasmacytoid dendritic cell neoplasm, Burkitt's lymphoma, diffuse large B-cell lymphoma, T-cell lymphoma, follicular lymphoma, hairy cell leukemia, small-cell or large-cell follicular lymphoma, malignant lymphoproliferative disorders, MALT lymphoma, mantle cell lymphoma, marginal zone lymphoma, multiple myeloma, myelodysplasia and myelodysplastic syndrome, Hodgkin's lymphoma, plasmablastic lymphoma, plasmacytoid dendritic cell neoplasm, B-cell lymphoma, and Waldenstrom's macroglobulinemia.

As used herein, the term "glypican-3 (GPC3)" is a heparan sulfate (HS) glycoprotein belonging to the heparan sulfate proteoglycan family, which is anchored to the cell membrane surface by glycosylphosphatidylinositol (GPI). As used herein, "GPC3" includes mature or immature fulllength wild-type GPC3 protein or mutants thereof (for example, point mutations, insertion mutations, or deletion mutations), splice variants, orthologs, and fragments of the foregoing GPC3. By way of example, as used herein, "GPC3" may be derived from mammals, for example, humans, primates such as monkeys (for example, rhesus monkeys and cynomolgus monkeys), and rodents, for example, mice and rats.

As used herein, the term "hinge region" may be considered as a portion that provides flexibility to the extracellular antigen-binding region, and is generally used to maintain further stable expression and activity of the chimeric antigen receptor in the immune effector cell. The hinge region may be derived from the extracellular domain of CD8α or CD28, or from the hinge region of IgG.

As used herein, the term "signal peptide" refers to a polypeptide linked to the N-terminus of the chimeric antigen receptor, which may affect the efficiency of protein secretion. The signal peptide may be derived from a cell adhesion molecule, for example, the signal peptide of CD8, or from the immunoglobulin heavy chain signal peptide of an immune effector cell, for example, a T cell or an NK cell.

As used herein, the term "polypeptide" refers to a compound composed of amino acid residues covalently linked by peptide bonds. Polypeptides include, for example, biologically active fragments, substantially homologous polypeptides, homodimers, heterodimers, variants of polypeptides, modified polypeptides, derivatives, analogs, fusion proteins, and the like. Polypeptides include natural peptides, recombinant peptides, synthetic peptides, or combinations thereof.

As used herein, the term "isolated" refers to something obtained from its natural state by artificial means. If a certain "isolated" substance or component occurs in nature, then it is possible that its natural environment has been altered, or that the substance has been separated from its natural environment, or both. For example, a certain polynucleotide or polypeptide that naturally exists in a living animal and that has not been isolated, when separated from such natural state in a highpurity form of the same polynucleotide or polypeptide, is referred to as "isolated." The term "isolated" does not exclude the presence of admixed artificial or synthetic substances, nor does it exclude the presence of other impurities that do not affect the activity of the substance.

As used herein, the term "encoding" refers to the inherent property of a specific nucleotide sequence in a polynucleotide (such as a gene, cDNA, or mRNA) to serve, in biological processes, as a template for the synthesis of other polymers and macromolecules having a defined nucleotide sequence (for example, rRNA, tRNA, and mRNA) or a defined amino acid sequence, and the biological properties resulting therefrom. Therefore, if the transcription and translation of the mRNA corresponding to a gene produce a protein in a cell or other biological system, the gene encodes the protein. Both the coding strand (whose nucleotide sequence is identical to the mRNA sequence and is typically provided in a sequence listing) and the non-coding strand (which is used as the template for transcription of the gene or cDNA) may be referred to as encoding the protein, or the other products of the gene or cDNA.

As used herein, the term "vector" refers to a construct capable of delivering, and preferably expressing, one or more genes or sequences of interest in a host cell. Examples of vectors include, but are not limited to, viral vectors, naked DNA or RNA expression vectors, plasmids, cosmid or bacteriophage vectors, DNA or RNA expression vectors associated with cationic condensing agents, DNA or RNA expression vectors encapsulated in liposomes, and certain eukaryotic cells, such as producer cells. A "vector" may comprise "regulatory elements" to regulate the expression of a gene or sequence.

As used herein, the term "regulatory element" includes a promoter (for example, a constitutive promoter or an inducible promoter), an enhancer (for example, a 35S promoter or a 35S enhanced promoter), an internal ribosome entry site (IRES), and other expression control elements (for example, transcription termination signals such as polyadenylation signals and poly-U sequences). In some cases, regulatory elements include those sequences that direct the constitutive expression of a nucleotide sequence in many types of cells, as well as those sequences that direct the expression of the nucleotide sequence only in certain cells (for example, tissue-specific regulatory sequences). A tissue-specific promoter may direct expression primarily in a desired tissue of interest, such as muscle, neurons, bone, skin, blood, a specific organ (for example, liver or pancreas), or a particular cell type (for example, lymphocytes). In some cases, regulatory elements may also direct expression in a time-dependent manner (for example, in a cell cycle-dependent or developmental stage-dependent manner), which may or may not be tissue- or cell-type specific.

As used herein, the term "immune effector cell" refers to an immune cell that is capable of participating in the clearance of foreign antigens and performing effector functions in an immune response, and that may be used as an autologous cell or an allogeneic cell for cellular immunotherapy in cell therapy.

As used herein, the term "effective amount" refers to an amount that provides a therapeutic or prophylactic benefit.

The present disclosure is further illustrated below by way of examples; however, the present disclosure is not thereby limited to the scope of the examples. In the following examples, experimental methods for which specific conditions are not indicated were conducted according to conventional methods and conditions, or according to product instructions. The reagents and starting materials used in the present disclosure are all commercially available.

### Example 1

This example describes the preparation of mutant CAR-T (CAR-T-1) cells, in which all lysine (K) sites of the intracellular segment of CAR-T-1 were mutated to glycine (G), comprising the following steps.
1) Construction of the CAR plasmid. In this example, a third-generation CAR targeting GPC3 was used. The coding sequence of the mutant CAR (CAR-1) is set forth in SEQ ID NO: 1, and the amino acid sequence is set forth in SEQ ID NO: 2. The mutant CAR comprises, in order, the following parts: a CD8α signal peptide (coding sequence set forth in SEQ ID NO: 3; amino acid sequence set forth in SEQ ID NO: 4), an hGPC3 scFv (YP7) (coding sequence set forth in SEQ ID NO: 5; amino acid sequence set forth in SEQ ID NO: 6), a CD8α hinge region (coding sequence set forth in SEQ ID NO: 7; amino acid sequence set forth in SEQ ID NO: 8), a CD8α transmembrane region (coding sequence set forth in SEQ ID NO: 9; amino acid sequence set forth in SEQ ID NO: 10), a mutant CD28 costimulatory domain (coding sequence set forth in SEQ ID NO: 11; amino acid sequence set forth in SEQ ID NO: 12), a mutant 4-1BB costimulatory domain (coding sequence set forth in SEQ ID NO: 13; amino acid sequence set forth in SEQ ID NO: 14), and a mutant CD3ζ intracellular domain (coding sequence set forth in SEQ ID NO: 15; amino acid sequence set forth in SEQ ID NO: 16). The above CAR-1 coding sequence was cloned into the pHAGE-EF1αL-eGFP vector (Addgene, Cat. No. 126686) and verified by sequencing.
2) Construction of the CAR lentiviral vector. In this example, a second-generation lentiviral packaging system was used, and the plasmid mixture was prepared at the following ratio: psPAX2 (Addgene, Cat. No. 12260) : pMD2.G (Addgene, Cat. No. 12259) : CAR plasmid = 3 : 1 : 4. After mixing, the mixture was added dropwise to a 293T cell culture dish. Virus was collected at 48 h and 72 h, respectively, and a concentrated virus solution was obtained by ultrafiltration centrifugation, thereby providing the CAR lentiviral vector.
3) Preparation of CAR-T-1 cells. T cells were obtained from PBMCs from healthy human donors by magnetic bead sorting (Stemcell, Cat. No. 17951), and the T cells were activated using TransAct (Miltenyi, Cat. No. 130-111-160). After 1 day, the CAR lentiviral vector from step 2) was added for infection to obtain CAR-T-1 cells.

### Example 2

This example describes the preparation of mutant CAR-T (CAR-T-2) cells, and differs from Example 1 in that only all lysine (K) sites of the CD3ζ of the CAR were mutated to glycine (G). The amino acid sequence of the CAR in CAR-T-2 is set forth in SEQ ID NO: 17.

### Comparative Example 1

This comparative example describes the preparation of non-mutant CAR-T (CAR-T-NT) cells, mutant CAR-T-3 cells (in which all lysine (K) sites of the intracellular segment of the CAR were mutated to arginine (R)), mutant CAR-T-4 cells (in which all lysine (K) sites of the intracellular segment of the CAR were mutated to histidine (H)), and mutant CAR-T-5 cells (in which all lysine (K) sites of the intracellular segment of the CAR were mutated to glutamic acid (E)). This comparative example differs from Example 1 only in that the CAR coding sequence is different, such that all lysine (K) sites of the intracellular segment of the CAR are not mutated or are mutated to the corresponding amino acid. The amino acid sequences of the CAR in non-mutant CAR-T, mutant CAR-T-3, mutant CAR-T-4, and mutant CAR-T-5 are set forth in SEQ ID NOs: 18, 19, 20, and 21, respectively.

This comparative example also describes the preparation of Mock cells, which are wild-type T cells that were not infected with lentivirus. T cells were obtained from PBMCs from healthy human donors by magnetic bead sorting (Stemcell, Cat. No. 17951), and the T cells were activated using TransAct (Miltenyi, Cat. No. 130-111-160), thereby providing the Mock cells.

### Example 3: Detection of surface CAR Degradation Rate of T Cells

This example detects the antigen stimulation-mediated CAR degradation level of each of the GPC3 CAR-T cells, namely CAR-T-NT cells, CAR-T-1 cells, CAR-T-2 cells, CAR-T-3 cells, CAR-T-4 cells, and CAR-T-5 cells, in vitro.

The GPC3 CAR-T cells and the GPC3-positive target cells Hep-G2 were co-cultured in a 24-well cell culture plate at an effector-to-target ratio of 1:4. Three replicate wells were set up for each type of CAR-T cells. At 0 h, 24 h, 48 h, 72 h, and 96 h, a portion of T cells in the culture supernatant was collected, and the surface CAR expression level of CAR-T cells was detected by flow cytometry.

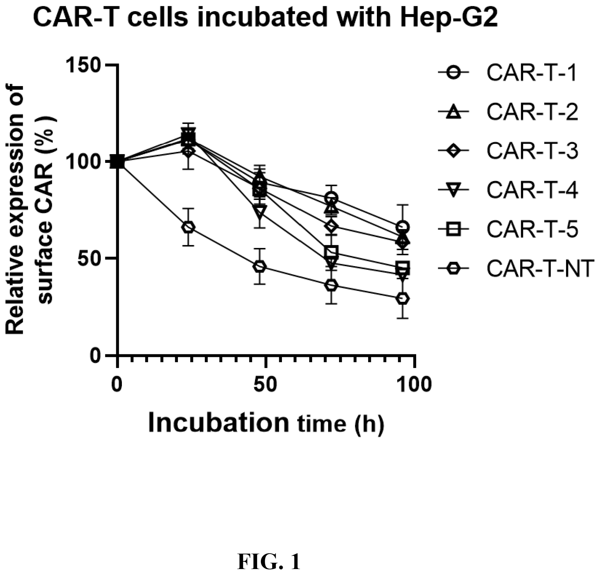

The results are shown in FIG. 1, in which the values displayed on the horizontal axis represent the relative CAR expression level (the CAR expression value at 0 h being defined as 1). After the CAR-T cells were allowed to act on the target cells, the surface CAR expression level of CAR-T-NT cells rapidly decreased, whereas the surface CAR expression level of CAR-T-1 cells, CAR-T-2 cells, CAR-T-3 cells, CAR-T-4 cells, and CAR-T-5 cells exhibited a brief increase followed by a decrease. Compared with the CAR-T-NT group, the extent of the decrease in surface CAR expression level was smaller in CAR-T-1 cells, CAR-T-3 cells, CAR-T-4 cells, and CAR-T-5 cells, which all had mutations of lysines in the intracellular domain of the CAR; among these, CAR-T-1, in which all lysine (K) sites of the intracellular domain of the CAR were mutated to glycine (G), exhibited the smallest decrease. At 96 h of co-culture, the relative average surface CAR expression level was 66.19%, whereas that of the CAR-T-NT group was only 29.47%. In addition, it was found in the experiment that the extent of the decrease in surface CAR expression level of CAR-T-2 cells, in which only the lysine (K) sites in the CD3ζ region were mutated to glycine (G), was also significantly lower than that of the CAR-T-NT group, indicating that mutation of only the CD3ζ lysine (K) sites of the CAR to glycine (G) is still able to significantly slow down the downregulation of CAR expression level.

### Example 4: In Vitro Tumor Cell Killing Activity Detection of T Cells

This example detects the in vitro tumor cell killing activity of Mock cells, CAR-T-NT cells, CAR-T-1 cells, CAR-T-2 cells, CAR-T-3 cells, CAR-T-4 cells, and CAR-T-5 cells.

The T cells were seeded in a 96-well U-bottom cell culture plate together with Hep-G2 cells expressing luciferase at three different effector-to-target ratios (1:2, 1:1, and 2:1). After 16 h of culture, a luciferase substrate was added, and readings were obtained using a microplate reader to detect the in vitro killing activity. A group of Hep-G2 cells expressing luciferase without seeded T cells was used as the negative control group. The average value of the negative control group was recorded as a, and the value of the experimental well was b; the percentage killing value of the experimental well (killing %) = (1 - b/a) × 100%.

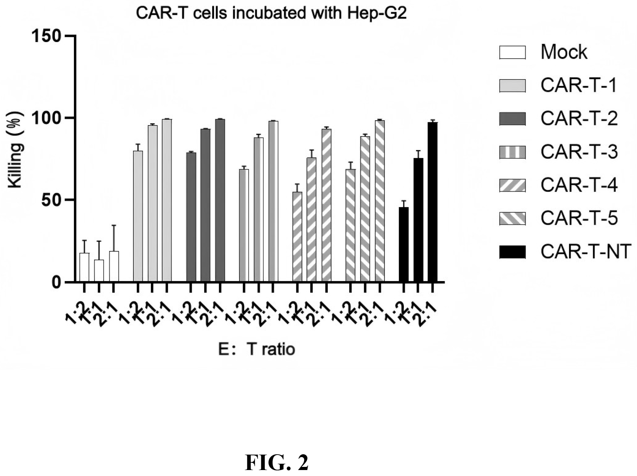

The results are shown in FIG. 2. There was no significant difference in the killing effect of the CAR-T cells of all groups on the target cells at a high effector-to-target ratio (2:1). However, at a low effector-to-target ratio (1:2), the killing ratio of CAR-T-NT was only 45.58%; correspondingly, the killing ratios were 80.17% for CAR-T-1, 79.08% for CAR-T-2, 68.72% for CAR-T-3, 54.87% for CAR-T-4, and 68.94% for CAR-T-5. The results indicate that, at a low effector-to-target ratio (1:2), compared with the non-mutant CAR-T-NT cells, mutating all lysine (K) sites of the intracellular domain of the CAR to glycine (G), arginine (R), histidine (H), or glutamic acid (E) all enhanced the tumor cell killing activity of the CAR-T cells. Mutation of all lysine (K) sites of the intracellular domain of the CAR to glycine (G) provided the greatest enhancement of tumor cell killing activity. It was also found in the experiment that the tumor cell killing activity of the CAR-T-2 cell group, in which only the lysine (K) sites in the CD3ζ region were mutated to glycine (G), was also significantly greater than that of the CAR-T-NT group at a low effector-to-target ratio (1:2), indicating that mutation of only the CD3ζ lysine (K) sites of the CAR to glycine (G) is still able to significantly enhance the tumor cell killing activity of CAR-T.

### Example 5: In Vivo Antitumor Efficacy Detection of T Cells in Tumor-Bearing Mice

This example detects the in vivo antitumor efficacy of CAR-T-NT cells, CAR-T-1 cells, CAR-T-2 cells, CAR-T-3 cells, CAR-T-4 cells, and CAR-T-5 cells in a tumor-bearing mouse model.

A liver cancer intraperitoneal model was established in severely immunodeficient N-BDG mice using Hep-G2 cells carrying luciferase. Six days after model establishment, the mice were randomly divided into groups based on the results of in vivo imaging, namely the Mock group, CAR-T-NT group, CAR-T-1 group, CAR-T-2 group, CAR-T-3 group, CAR-T-4 group, and CAR-T-5 group, with 5 mice in each group. On Day 7 after model establishment, the mice were administered the respective cells. The Mock group was injected with wild-type T cells that had not been infected with lentivirus, and the other experimental groups were injected with CAR-T cells (the CAR-T cell injection dose was calculated based on positive CAR-T cells, and the same dose of positive CAR-T cells was injected). The day of administration was recorded as Day 0. In vivo imaging was performed every 1 week, and body weight changes were recorded every 3 days to observe tumor growth and the health indicators of the mice.

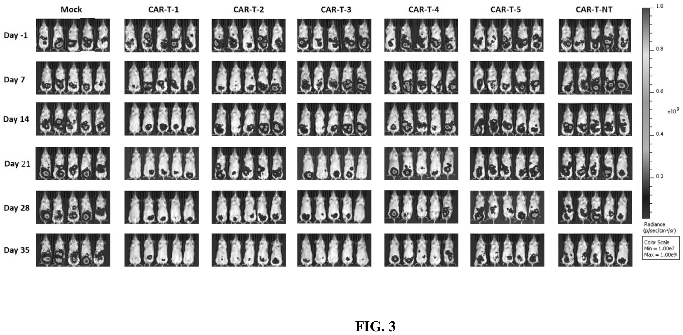

The results are shown in FIG. 3. Compared with the Mock group, the tumors in the CAR-T-NT group, CAR-T-1 group, CAR-T-2 group, CAR-T-3 group, CAR-T-4 group, and CAR-T-5 group were all inhibited to a certain extent. Compared with the non-mutant CAR-T-NT cells, mutating all lysine (K) sites of the intracellular domain of the CAR to glycine (G), arginine (R), histidine (H), or glutamic acid (E) all enhanced the tumor cell killing activity of the CAR-T cells and further enhanced the tumor inhibition effect. Among these, mutation of all lysine (K) sites of the intracellular domain of the CAR to glycine (G) provided the best tumor inhibition effect, and complete tumor elimination was achieved in 2 mice of the CAR-T-1 group. It was also found in the experiment that the tumor inhibition effect of the CAR-T-2 cell group, in which only the lysine (K) sites in the CD3ζ region were mutated to glycine (G), was also significantly greater than that of the CAR-T-NT group, indicating that mutation of only the CD3ζ lysine (K) sites of the CAR to glycine (G) is still able to significantly enhance the tumor inhibition effect of CAR-T.

### Example 6

This example describes the preparation of mutant CAR-T cells (CAR-T-GC33-1 and CAR-T-1-B1-1). The difference from CAR-T-1 in Example 1 is that the antibody YP7 in the CAR was replaced with GC33 (the amino acid sequence of which is set forth in SEQ ID NO: 22) or 1-B1 (the amino acid sequence of which is set forth in SEQ ID NO: 23), thereby obtaining CAR-T-GC33-1 cells and CAR-T-1-B1-1 cells.

This example also describes the preparation of non-mutant CAR-T cells (CAR-T-GC33-NT and CAR-T-1-B1-NT). The difference from the CAR-T-NT cells in Comparative Example 1 is that the antibody YP7 in the CAR was replaced with GC33 (the amino acid sequence of which is set forth in SEQ ID NO: 22) or 1-B1 (the amino acid sequence of which is set forth in SEQ ID NO: 23), thereby obtaining CAR-T-GC33-NT cells and CAR-T-1-B1-NT cells.

### Example 7

This example detects the in vitro tumor cell killing activity of CAR-T-GC33-1 cells, CAR-T-1-B1-1 cells, CAR-T-GC33-NT cells, and CAR-T-1-B1-NT cells of Example 6.

The above CAR-T cells were seeded in a 96-well U-bottom cell culture plate together with Hep-G2 cells expressing luciferase at an effector-to-target ratio of 1:2. After 16 h of culture, a luciferase substrate was added, and readings were obtained using a microplate reader to detect the in vitro killing activity. A group of Hep-G2 cells expressing luciferase without seeded T cells was used as the negative control group. The average value of the negative control group was recorded as a, and the value of the experimental well was b; the percentage killing value of the experimental well (killing %) = (1 - b/a) × 100%.

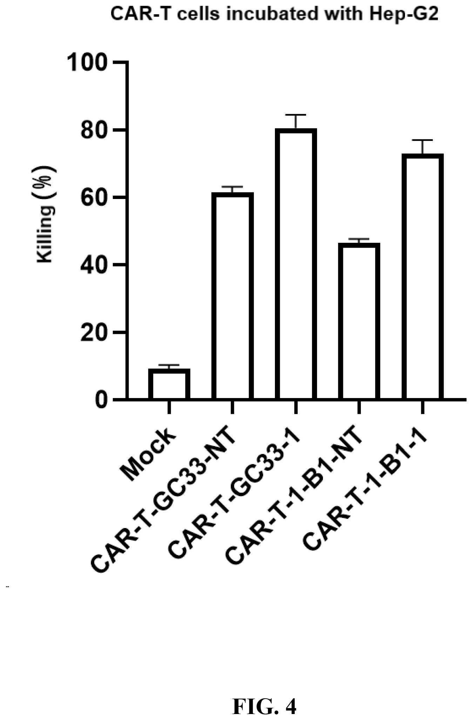

The results are shown in FIG. 4. At an effector-to-target ratio of 1:2, the killing ratio of CAR-T-GC33-1 cells was much higher than that of CAR-T-GC33-NT cells, and the killing ratio of CAR-T-1-B1-1 cells was much higher than that of CAR-T-1-B1-NT cells. These results indicate that, compared with CAR-T cells in which the CD28 costimulatory domain, 4-1BB costimulatory domain, and CD3ζ intracellular domain of the CAR intracellular domain were not mutated, mutation of the CD28 costimulatory domain, 4-1BB costimulatory domain, and CD3ζ intracellular domain of the CAR intracellular domain to glycine (G) can greatly enhance the tumor cell killing activity.

### Example 8

This example describes the preparation of mutant CAR-T cells (CAR-T-GC33-2 and CAR-T-1-B1-2). The difference from CAR-T-2 in Example 2 is that the antibody YP7 in the CAR was replaced with GC33 (the amino acid sequence of which is set forth in SEQ ID NO: 22) or 1-B1 (the amino acid sequence of which is set forth in SEQ ID NO: 23), thereby obtaining CAR-T-GC33-2 cells and CAR-T-1-B1-2 cells.

This example also describes the preparation of non-mutant CAR-T cells (CAR-T-GC33-NT and CAR-T-1-B1-NT). The difference from the CAR-T-NT cells in Comparative Example 1 is that the antibody YP7 in the CAR was replaced with GC33 (the amino acid sequence of which is set forth in SEQ ID NO: 22) or 1-B1 (the amino acid sequence of which is set forth in SEQ ID NO: 23), thereby obtaining CAR-T-GC33-NT cells and CAR-T-1-B1-NT cells.

### Example 9

This example detects the in vitro tumor cell killing activity of CAR-T-GC33-2 cells and CAR-T-1-B1-2 cells of Example 8, and CAR-T-GC33-NT cells and CAR-T-1-B1-NT cells of Example 6.

The above CAR-T cells were seeded in a 96-well U-bottom cell culture plate together with Hep-G2 cells expressing luciferase at an effector-to-target ratio of 1:2. After 16 h of culture, a luciferase substrate was added, and readings were obtained using a microplate reader to detect the in vitro killing activity. A group of Hep-G2 cells expressing luciferase without seeded T cells was used as the negative control group. The average value of the negative control group was recorded as a, and the value of the experimental well was b; the percentage killing value of the experimental well (killing %) = (1 - b/a) × 100%.

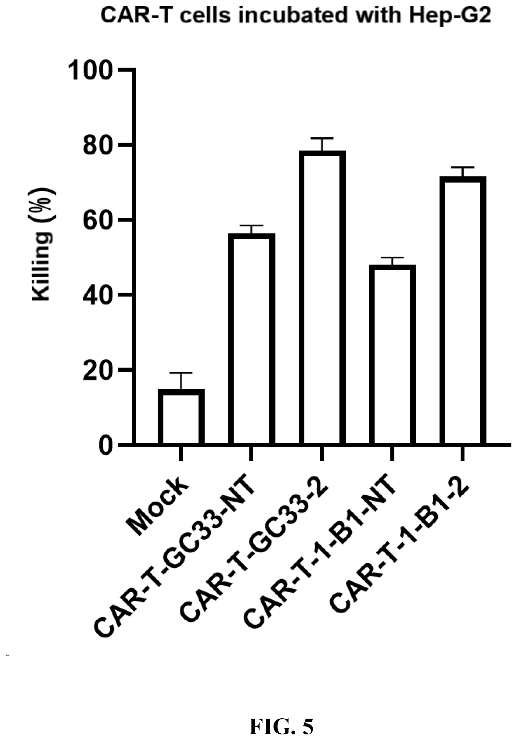

The results are shown in FIG. 5. At an effector-to-target ratio of 1:2, the killing ratio of CAR-T-GC33-2 cells was much higher than that of CAR-T-GC33-NT cells, and the killing ratio of CAR-T-1-B1-2 cells was much higher than that of CAR-T-1-B1-NT cells. These results indicate that, compared with CAR-T cells in which the CD3ζ region of the CAR intracellular domain was not mutated, mutation of the lysine (K) sites in the CD3ζ region of the CAR intracellular domain to glycine (G) can greatly enhance the tumor cell killing activity.

## Claims

1. A chimeric antigen receptor, comprising an extracellular domain, a transmembrane domain, and an intracellular domain connected in sequence, wherein
the extracellular domain recognises and binds to an antigen;
the intracellular domain comprises an activation domain, or comprises a costimulatory domain and an activation domain; and
relative to a parental intracellular domain, lysines in the costimulatory domain and/or the activation domain are partially or completely substituted with glycines.

2. The chimeric antigen receptor according to claim 1, wherein the chimeric antigen receptor comprises at least one costimulatory domain; and
preferably, relative to the parental intracellular domain, lysines in the activation domain are partially or completely substituted with glycines; or, relative to the parental intracellular domain, lysines in at least one of the costimulatory domain and the activation domain are partially or completely substituted with glycines.

3. The chimeric antigen receptor according to claim 1 or 2, wherein
the activation domain comprises a functional signalling domain of a protein selected from the group consisting of CD3ζ, CD3γ, CD3δ, CD3ε, FcεRIγ, FcRβ, CD79a, CD79b, FcγRIIa, DAP10, and/or DAP12; preferably CD3ζ;
and/or the costimulatory domain is a costimulatory domain of a protein selected from the group consisting of 4-1BB, CD27, CD28, OX-40, and ICOS; preferably CD28 and/or 4-1BB;
and/or the transmembrane domain is a transmembrane domain of a protein selected from the group consisting of CD28, CD3e, CD45, CD4, CD5, CD8, CD9, CD16, CD22, CD33, CD37, CD64, CD80, CD86, CD134, CD137, and/or CD154; preferably CD8;
and/or the extracellular domain is an antibody or an antigen-binding fragment, for example an scFv, a single-domain antibody, or an F(ab)'.

4. The chimeric antigen receptor according to any one of claims 1 to 3, wherein
the chimeric antigen receptor further comprises a hinge region, wherein the hinge region connects the extracellular domain and the transmembrane domain; preferably, the hinge region comprises a hinge region of CD8α or CD28, for example CD8α;
and/or the chimeric antigen receptor further comprises a signal peptide, preferably a signal peptide of CD8α;
and/or the extracellular domain targets a tumour antigen, preferably, the tumour antigen is selected from the group consisting of GPC3, CLDN18.2, GCC, EGFRvIII, ROR1, CLDN6, MSLN, ALPP, MUC1, LGR5, HER2, OR2H1, DLL-3, C-MET, glyco-cMET, glyco-LAMP1, CD123, CD33, CLL-1, CD70, CD38, FLT3, and GRP78.

5. The chimeric antigen receptor according to any one of claims 1 to 4, wherein
the activation domain is a functional signalling domain of CD3ζ, and the activation domain preferably comprises an amino acid sequence set forth in SEQ ID NO: 16;
and/or the costimulatory domain is a costimulatory domain of CD28, and the costimulatory domain preferably comprises an amino acid sequence set forth in SEQ ID NO: 12 and/or SEQ ID NO: 14;
and/or the transmembrane domain is a transmembrane domain of CD8, and the transmembrane domain preferably comprises an amino acid sequence set forth in SEQ ID NO: 10;
and/or the hinge region is a hinge region of CD8α, and preferably comprises an amino acid sequence set forth in SEQ ID NO: 8;
and/or the signal peptide is a signal peptide of CD8α, and preferably comprises an amino acid sequence set forth in SEQ ID NO: 4.

6. A polypeptide, wherein the polypeptide comprises the chimeric antigen receptor according to any one of claims 1 to 5;
preferably, the polypeptide is further linked to an additional functional molecule;
more preferably, the additional functional molecule is selected from the group consisting of a signal peptide, a protein tag, other antigen-binding molecules, a cytokine, and combinations thereof.

7. An isolated nucleic acid, wherein the nucleic acid encodes the chimeric antigen receptor according to any one of claims 1 to 5 or the polypeptide according to claim 6;
preferably, a nucleic acid sequence encoding the transmembrane domain of the chimeric antigen receptor is set forth in SEQ ID NO: 9; and/or a nucleic acid sequence encoding the activation domain of the chimeric antigen receptor is set forth in SEQ ID NO: 15; and/or a nucleic acid sequence encoding the costimulatory domain of the chimeric antigen receptor is set forth in SEQ ID NO: 11 and/or SEQ ID NO: 13; and/or a nucleic acid sequence encoding the hinge region of the chimeric antigen receptor is set forth in SEQ ID NO: 7; and/or a nucleic acid sequence encoding the signal peptide of the chimeric antigen receptor is set forth in SEQ ID NO: 3.

8. A vector, wherein the vector comprises the nucleic acid according to claim 7;
preferably, the vector is a plasmid, a cosmid, a bacteriophage, or a viral vector;
more preferably, a backbone of the viral vector is pHAGE.

9. A gene expression cassette, wherein the gene expression cassette comprises a promoter and the nucleic acid according to claim 7.

10. An immune effector cell, wherein the immune effector cell expresses the chimeric antigen receptor according to any one of claims 1 to 5 or comprises the polypeptide according to claim 6;
preferably, the immune effector cell is selected from one or more of T cells, NK cells, NKT cells, monocytes, mast cells, macrophages, dendritic cells, CIK cells, and stem cell-derived immune effector cells;
more preferably, the immune effector cell is a T cell, an NK cell, or an NKT cell; for example, a T cell.

11. A method for preparing an immune effector cell, wherein the method comprises a step of introducing the vector according to claim 8 or the gene expression cassette according to claim 9 into a parental immune effector cell, thereby obtaining the immune effector cell.

12. A pharmaceutical composition, wherein the pharmaceutical composition comprises the chimeric antigen receptor according to any one of claims 1 to 5, the polypeptide according to claim 6, the nucleic acid according to claim 7, the vector according to claim 8, the gene expression cassette according to claim 9, and/or the immune effector cell according to claim 10;
preferably, the pharmaceutical composition further comprises a pharmaceutically acceptable carrier and/or excipient.

13. The chimeric antigen receptor according to any one of claims 1 to 5, the polypeptide according to claim 6, the nucleic acid according to claim 7, the vector according to claim 8, the gene expression cassette according to claim 9, the immune effector cell according to claim 10, and/or the pharmaceutical composition according to claim 12, for use in a manufacture of a medicament for diagnosis, prevention and/or treatment of a tumour or cancer;
wherein the tumour comprises a solid tumour or a non-solid tumour;
preferably, the solid tumour is selected from the group consisting of liver cancer, gastric cancer, lung cancer, breast cancer, head and neck cancer, bladder cancer, ovarian cancer, cervical cancer, kidney cancer, pancreatic cancer, liposarcoma, melanoma, adrenal cancer, schwannoma, malignant fibrous histiocytoma, and oesophageal cancer; and the non-solid tumour is selected from the group consisting of B-cell lymphoma, Hodgkin's lymphoma, chronic myeloid leukaemia, and acute myeloid leukaemia.

14. A method for diagnosis, prevention and/or treatment of a tumour or cancer in a patient in need thereof, wherein the method comprises administering to the patient in need thereof an effective amount of the chimeric antigen receptor according to any one of claims 1 to 5, the polypeptide according to claim 6, the nucleic acid according to claim 7, the vector according to claim 8, the gene expression cassette according to claim 9, the immune effector cell according to claim 10, and/or the pharmaceutical composition according to claim 12;
wherein the tumour comprises a solid tumour or a non-solid tumour;
preferably, the solid tumour is selected from the group consisting of liver cancer, gastric cancer, lung cancer, breast cancer, head and neck cancer, bladder cancer, ovarian cancer, cervical cancer, kidney cancer, pancreatic cancer, liposarcoma, melanoma, adrenal cancer, schwannoma, malignant fibrous histiocytoma, and oesophageal cancer; and the non-solid tumour is selected from the group consisting of B-cell lymphoma, Hodgkin's lymphoma, chronic myeloid leukaemia, and acute myeloid leukaemia.

15. A composition for diagnosis, prevention and/or treatment of a tumour or cancer, wherein the composition comprises the chimeric antigen receptor according to any one of claims 1 to 5, the polypeptide according to claim 6, the nucleic acid according to claim 7, the vector according to claim 8, the gene expression cassette according to claim 9, the immune effector cell according to claim 10, and/or the pharmaceutical composition according to claim 12;
wherein the tumour comprises a solid tumour or a non-solid tumour;
preferably, the solid tumour is selected from the group consisting of liver cancer, gastric cancer, lung cancer, breast cancer, head and neck cancer, bladder cancer, ovarian cancer, cervical cancer, kidney cancer, pancreatic cancer, liposarcoma, melanoma, adrenal cancer, schwannoma, malignant fibrous histiocytoma, and oesophageal cancer; and the non-solid tumour is selected from the group consisting of B-cell lymphoma, Hodgkin's lymphoma, chronic myeloid leukaemia, and acute myeloid leukaemia.
